# EUROPÄISCHE PATENTSCHRIFT

(11) **EP 1 687 014 B1**
(45) Veröffentlichungstag und Bekanntmachung des Hinweises auf die Patenterteilung: **02.05.2007**
(21) Anmeldenummer: 04818400.6
(22) Anmeldetag: 12.11.2004
(51) Int. Cl.: A61K 36/00, A23L 1/30, A61P 3/08, A61P 3/10

(54) **VERWENDUNG EINER ZUSÄTZLICH FERMENTIERTEN GETREIDESCHLEMPE ZUR VORBEUGUNG UND/ODER BEHANDLUNG ERHÄHTER BLUTZUCKER-WERTE**
USE OF ADDITIONALLY FERMENTED DISTILLERS GRAINS FOR PREVENTING AND/OR TREATING INCREASED BLOOD SUGAR VALUES
UTILISATION DE DRECHE DE DISTILLERIE FERMENTEE POUR PREVENIR ET/OU TRAITER DES TAUX AUGMENTES DE GLYCEMIE

(30) Priorität: 12.11.2003 DE 10352822
(43) Veröffentlichungstag der Anmeldung: 09.08.2006
(73) Patentinhaber: BIOGHURT BIOGARDE GMBH & CO. KG, 85354 Freising (DE)
(72) Erfinder: ZIRZOW, Karl-Heinz, 84104 Rudelzhausen (DE); BÖKENKAMP, Dirk, 59368 Werne (DE); MÜLLER, Martin, 59494 Soest (DE); TOM DIECK, Heike, 85354 Freising (DE)
(74) Vertreter: Dey, Michael
(86) Internationale Anmeldenummer: PCT/EP2004/012875
(87) Internationale Veröffentlichungsnummer: WO 2005/046705

(56) Entgegenhaltungen:
- WO-A-01/10245
- DE-A1- 3 904 962
- US-A- 5 006 337

## Beschreibung

Gegenstand der vorliegenden Erfindung ist die Verwendung einer mit einer Joghurt- und/oder Butterei-Kultur fermentierten Getreideschlempe zur Herstellung einer Zusammensetzung zur Vorbeugung und/oder Behandlung erhöhter Blutzucker-Werte.

Getreideschlempen enthalten zahlreiche ernährungsphysiologisch bedeutungsvolle Komponenten, wie Restkohlenhydrate, Fette, Ballaststoffe usw., so dass sie seit langem als Futtermittel in der Tierzucht und insbesondere in der Schweinemast eine bedeutende Rolle spielen.

Speziell die in den mit Hefekulturen fermentierten ("angemaischten") Getreideschlempen noch enthaltenen Ballaststoffe und Vitamine machen diesen ursprünglichen "Abfallstoff" zu einem wertvollen Sekundärrohstoff, nicht zuletzt auch für die menschliche Ernährung.

So ist aus der WO 01/10 245 ein Verfahren zur Herstellung von Lebensmitteln, diätetischen Lebensmitteln und Lebensmittelzusätzen bekannt, welches von einer direkt dem Brennkessel entnommenen Getreideschlempe, also einer mit Hefen fermentierten Schlempe, ausgeht. Nachdem die Getreideschlempe eingedickt, aber nicht getrocknet wurde, wird sie mit Milch und einer Joghurt- und/oder Butterei-Kultur ein weiteres mal fermentiert. Dabei werden im Wesentlichen vier aufeinanderfolgende Verfahrensschritte durchgeführt, wobei zunächst
a) die Getreideschlempe in einem speziellen Druckbereich auf ca. 15 bis 17 % TM (Trockenmasse) eingedickt wird, dann
b) diese mit einer mit den Joghurt- oder Butterei-Kulturen angeimpften Milch vermischt, und anschließend
c) entweder mit den Joghurt-Kulturen bei 38 bis 48 °C für 10 bis 20 Stunden oder mit einer Butterei-Kultur bei 18 bis 24 °C bis zu 36 Stunden lang fermentiert wird; abschließend wird
d) dieses Vorprodukt mit weiterer Milch oder einem Milchkonzentrat vermischt auf unter 15 °C abgekühlt; ggf. folgt noch ein zusätzlicher Sprühtrocknungsschritt, um zu einem pulverförmigen und lagerfähigen Produkt zu gelangen.

Die mit diesem Verfahren sowohl als stabiles Flüssigprodukt als auch als lagerfähiges Pulver erhältlichen Produkte stellen ernährungsphysiologisch wertvolle Vollkorn- und Eiweiß-Erzeugnisse dar, denen aufgrund der schonenden und zusätzlich durchgeführten Fermentation bei einem gleichbleibend hohen Ballaststoff-Anteil die Stärke-Anteile fast vollständig entzogen wurden, wobei ein Reststärkegehalt von höchstens 4 Gew.-% erzielt wird.

Im Zusammenhang mit den ernährungsphysiologischen Eigenschaften von Vollkorn und insbesondere von Vollkornreis wurde im Vergleich mit geschälten Produkten herausgefunden, dass sie in der Lage sind, die Insulin-Empfindlichkeit und die Blutfettwerte signifikant zu verbessern (Jang Y. et al: "Consumption of Whole Grain and Legume Powder reduces Insulin Demand, ..., in Patients with Coronary, Artery Disease: Randomized Controlled Clinical Trial"; Arteroscler. Thromb. Vasc. Biol. 21: 2065-2071, 2001).

Aus JP 101 46 166 sind physiologisch aktive Zusammensetzungen bekannt, die u.a. Brauereischlempen oder wässrige Extrakte daraus enthalten können. Diese ausschließlich mit Hefen fermentierten Schlempen werden zur Behandlung von Krebs und damit verbundenen Symptomen eingesetzt, wobei u.a. auch Diabetes und Fettleibigkeit erwähnt sind.

Gemäß JP 2002-371003 sollen konzentrierte Extrakte eines fermentierten Weizenmaterials zur Behandlung von Diabetes geeignet sein, wobei sie insbesondere den Anstieg des Blutzuckerspiegels verhindern sollen. Dieser anti-diabetische Effekt wurde an Maus-Populationen mit Hilfe eines ausschließlich mit Hefe fermentierten Weizenextraktes aufgezeigt.

Speziell der Diabetes Typ II und dessen verbundene (Vor-)Formen spielen in den Industrienationen, aber durch soziokulturelle Veränderungen zunehmend auch in Entwicklungsländern und in der jüngeren Population, bereits gegenwärtig, vor allem aber in Zukunft eine gesundheitspolitisch, aber auch volkswirtschaftlich bedeutende Rolle.

Bei Diabetes handelt es sich längst nicht mehr nur um eine harmlose Begleiterscheinung des Alterns, sondern er stellt eine ernstzunehmende Stoffwechselstörung dar, die zu lebensverkürzenden Folgeerkrankungen führt, wie z. B. Herzinfarkt, Schlaganfall, Nierenversagen, Erblindung oder die sogenannten "zuckerbedingten" Amputationen.

Aus volkswirtschaftlicher Sicht verursachen speziell diese durch Diabetes bedingten und gefürchteten Komplikationen hohe Kosten. Insbesondere gilt es, die sich im Diabetes Typ II manifestierende und diesem Syndrom um viele Jahre vorausgehende Insulin-Resistenz, die mit fortschreitendem Alter zunimmt, nicht nur frühzeitig zu erkennen, sondern auch zu behandeln, um so den typischen Diabetes-Symptomen vorzubeugen.

Für die vorliegende Erfindung hat sich deshalb die Aufgabe gestellt, eine Zusammensetzung bereitzustellen, die aufgrund ihrer insbesondere ernährungsphysiologischen Eigenschaften dazu geeignet ist, pathologisch erhöhten Blutzucker-Werten vorzubeugen und/oder bereits erhöhte Blutzucker-Werte zu senken. Dabei stand die Verwendung einer weitgehend natürlichen Zusammensetzung im Vordergrund, die leicht zugänglich ist und die nach Möglichkeit neben den eigentlich angestrebten Effekten noch andere positive Einflüsse auf den Stoffwechsel des Körpers auszuüben in der Lage ist.

Gelöst wurde diese Aufgabe durch die Verwendung einer Getreideschlempe, die zusätzlich zu einer Fermentierung mit Hefe auch mit einer Joghurt- und/oder Butterei-Kultur fermentiert wurde, zur Herstellung einer Zusammensetzung zur Vorbeugung und/oder Behandlung erhöhter Blutzucker-Werte.

Eine Getreideschlempe ist der nach der destillativen Abtrennung des Alkohols flüssige Rückstand aus der Vergärung kohlenhydrathaltiger Rohstoffe aus Getreide. Sie enthält die unvergärbaren Bestandteile des eingemaischten Rohmaterials und Rückstände der Hefezellen. Auf je 100 kg vergorenes Getreide entfallen ca. 500 1 Schlempe mit 5 bis 7% Trockenrückstand (ph 3,6 - 4,0). Der Trockenrückstand besteht etwa zur Hälfte aus löslichen, zur Hälfte aus unlöslichen Bestandteilen.

Überraschend hat sich bei der regelmäßigen erfindungsgemäßen Anwendung herausgestellt, dass erhöhte Blutzucker-Werte signifikant auf gleichbleibend erniedrigte Werte gebracht werden können.

Zur Beurteilung erhöhter Blutzucker-Werte wird zwischen Nüchtern- und/oder postprandialem (2-Stunden-)Blutzucker-Wert unterschieden. Bei Nüchtern-Blutzuckerwerten ≥ 110 mg/dl spricht man von einem erhöhten Blutzuckerwert, bei Nüchtern-Blutzuckerwerten ≥ 110 mg/dl und < 126 mg/dl liegt eine Prädiabetes-Form vor, und bei Nüchtern-Blutzuckerwerten ≥ 126 mg/dl liegt Diabetes vor. Bei postprandialen Blutzuckerwerten ≥ 140 mg/dl spricht man von einem erhöhten Blutzuckerwert, bei postprandialen Blutzuckerwerten ≥ 140 mg/dl und < 200 mg/dl liegt eine Prädiabetes-Form vor und bei postprandialen Blutzuckerwerten ≥ 200 mg/dl liegt Diabetes vor.

Als besonders geeignet hat sich im Zusammenhang mit der vorliegenden Erfindung die Verwendung einer mit Joghurt- und/oder Buttereikulturen vollständig fermentierten Getreideschlempe erwiesen, insbesondere bevorzugt einer Getreideschlempe, deren Reststärkegehalt maximal 4 Gewichts-%, bezogen auf das Gesamtgewicht der Getreideschlempe, beträgt.

Als bevorzugte Variante hat sich die Verwendung der erfindungsgemäßen Getreideschlempe zur Herstellung einer Zusammensetzung zur Vorbeugung und/oder Behandlung von Patienten mit Prädiabetes und/oder Diabetes und/oder einer Prädisposition dafür und damit zusammenhängender Formen und/oder Vorformen gezeigt. Dabei handelt es sich üblicherweise bevorzugt um Diabetes Typ IA und IB, Typ IIA und IIB und/oder autosomal vererbten Diabetes des Jugendalters.

Bei Prädiabetes handelt es sich um ein Stadium mit normaler Glucosetoleranz ohne klinische Symptome wie Hyperglykämie, Glucosurie etc., das dem Ausbruch eines Diabetes mellitus um Jahre vorausgehen kann. Die Indikation Prädiabetes beruht nicht zwingend auf einer retrospektiven Diagnose, sie bezieht sich allerdings meistens auf die Zeitspanne zwischen Konzeption und Erkennung des Diabetes.

Der Typ I Diabetes oder auch insulinabhängige Diabetes ist eine genetisch prädisponierte Diabetesform mit allmählicher Erschöpfung der körpereigenen Insulinsekretion bis hin zum absoluten Insulinmangel. Beim Typ IA, der praktisch im Kindesalter auftritt, wirken vermutlich virale Infekte manifestationsfördernd. Beim Typ IB des Erwachsenen, der bis zum 35. Lebensjahr auftritt, finden sich oft Inselzell-Antikörper im Serum Dieser Typ tritt auch gehäuft mit anderen Autoimmunerkrankungen auf. Die Therapie besteht in einer Diät sowie einer Insulinsubstitution.

Der Typ II Diabetes oder auch insulinunabhängige Diabetes tritt meist im höheren Lebensalter, gehäuft familiär auf und ist wahrscheinlich genetisch bedingt. Zum seltenen Typ IIA gehören Normalgewichtige, während die meisten Patienten zum Typ IIB gehören, welche übergewichtig sind. Beim Diabetestyp II liegt eine eingeschränkte, erhaltende oder zum Teil sogar erhöhte Insulinsekretion sowie verminderte Insulinempfindlichkeit der Gewebe und somit relativer Insulinmangel, bis hin zum absoluten Insulinmangel im späteren Stadium, vor. Daher ist im späten Stadium vom Typ II Diabetes eine Insulintherapie durchaus häufig anzutreffen. Therapiert wird dieser mit Gewichtsreduktion, Diät und eventuell oralen Antidiabetika. Der autosomal vererbte Diabetes des Jugendalters, auch MODY (Abkürzung für maturity onset diabetes of the young) hat einen unterschiedlichen, in der Regel klinisch milden Verlauf ohne Spätkomplikationen. Er ist eine eigenständige Diabetesform, die typischerweise vor dem 25. Lebensjahr auftritt und mit erhöhter Insulinsekretion, periphärer Insulinresistenz und Fettsucht einhergeht. Therapiert wird dieser Diabetestyp mit Gewichtsreduktion. Eine Therapie mit Insulin und oralen Antidiabetika ist nicht erforderlich, jedoch sind lebenslange Stoffwechselkontrollen nötig.

Die erfindungsgemäße Getreideschlempe ist zur Therapie der genannten Diabetestypen geeignet, da bei diesen eine Senkung der Blutzucker-Werte erforderlich bzw. eine möglichst geringe Belastung des Blutzuckerhaushalts angestrebt wird.

Des weiteren wird die Getreideschlempe erfindungsgemäß bevorzugt zur Vorbeugung und/oder Behandlung von mit Diabetes zusammenhängenden Formen und/oder Vorformen verwendet. Dazu zählen die sekundären Diabetesformen, potentieller Diabetes, latenter Diabetes, verminderte Glukosetoleranz und/oder klinisch manifester Diabetes.

Ein potentieller Diabetes liegt bei Personen mit nicht pathologischem oralem Glukose-Toleranztest vor, denen mit hoher Wahrscheinlichkeit durch familiäre Belastung ein Diabetes vorausgesagt werden kann.

Ein latenter Diabetes liegt bei Personen
1. mit normalen Werten beim oralen Glukose-Toleranztest vor, die unter Belastungssituationen wie Schwangerschaft, Infektion, Stress oder nach Gewichtszunahme (Adipositas) pathologische Werte aufweisen,
2. die bei Provokationstests pathologische Blutzuckerkurven zeigen.

Von einer verminderten Glukosetoleranz kann bei Personen
1. mit pathologischen Werten im oralen Glukose-Toleranztest, bei denen der Nüchtern-Blutzucker unter 110 mg/dl liegt,
2. die pathologische Werte im oralen Glukose-Toleranztest zeigen und bei denen die Nüchtern-Blutzucker-Werte ≥ 110 mg/dl und < 126 mg/dl betragen,
3. deren postprandiale Blutzucker-Werte ≥ 140 mg/dl und 200 mg/dl betragen, gesprochen werden.

Der klinisch manifeste Diabetes liegt bei Patienten mit pathologischen Blutzucker-Werten und Harnzucker-Ausscheidung sowie bei vorwiegend typischen Symptomen und eventuellen Komplikationen des Diabetes vor.

Zu den sekundären Diabetesformen zählen Pankreaserkrankungen, z.B. Hämochromatose, Endokrinopathien, z.B. Akromegalie, medikamentös bedingter Diabetes, z.B. Steroiddiabetes und andere Formen verminderter Glukosetoleranz und/oder Schwangerschaftsdiabetes. Bei dem Schwangerschaftsdiabetes wirkt die Schwangerschaft im Sinne einer Manifestationsförderung oder verschlechtert unter Umständen die Stoffwechsellage bei schon manifestem Diabetes mellitus. Sie ist eine insulinpflichtige Diabetesform. Nach der Geburt sinkt der Insulinbedarf deutlich. Da bei diesen mit Diabetes zusammenhängenden Formen und/oder Vorformen ebenfalls eine Senkung der erhöhten Blutzucker-Werte therapeutisch indiziert ist, ist die erfindungsgemäße Verwendung der fermentierten Getreideschlempe zur Vorbeugung und/oder Behandlung dieser Formen und/oder Vorformen geeignet.

Als besonders geeignet hat sich im Rahmen der vorliegenden Erfindung die Verwendung bei Patienten mit Diabetestyp II (Diabetestyp 2) und damit zusammenhängender Formen und/oder Vorformen gezeigt.

Insgesamt ist es ebenfalls möglich, die spezielle, fermentierte Getreideschlempe erfindungsgemäß zur Reduzierung des Körpergewichts, besonders bevorzugt zur gleichzeitigen Reduzierung des Körpergewichts zu verwenden, was sich vor allem in einem deutlich verbesserten Body-Mass-Index (BMI) von > 1 Einheit pro 4 Wochen äußert.

In diesem Zusammenhang berücksichtigt die vorliegende Erfindung eine bevorzugte Verwendungsvariante zur Herstellung einer Zusammensetzung zur Vorbeugung und/oder Behandlung von Patienten mit Adipositas und besonders bevorzugt zur Vorbeugung und/oder Behandlung von Patienten mit Adipositas und gleichzeitig erhöhten Nüchtern-Blutzuckerwerten. Mit der erfindungsgemäß verwendeten Getreideschlempe ist es möglich, diese adipositären Personen mit gleichzeitig erhöhten Nüchtern-Blutzucker-Werten schonend und ohne drastischen Eingriff in deren Ernährungsgewohnheiten erfolgreich zu behandeln, denn der Effekt des Produktes geht über den durch eine einfache Gewichtsabnahme hervorgerufenen Effekt hinaus.

Die Kombination dieser vorteilhaften Eigenschaften der verwendeten mehrfach fermentierten Getreideschlempe war so nicht vorherzusehen.

Die fermentierte Getreideschlempe betrifft im Rahmen der vorliegenden Erfindung somit bevorzugt sowohl die therapeutische als auch die nicht-therapeutische Anwendung.

Als weitere bevorzugte Variante kann die erfindungsgemäß verwendete Getreideschlempe zur Vorbeugung und/oder Behandlung von Patienten mit metabolischem Syndrom eingesetzt werden. Eine Person weist nach einer WHO-Definition (World Health Organization (1999), Definition, diagnosis and classification of diabetes mellitus and its complications. Part 1: Diagnosis and classification of diabetes mellitus. WHO: (WHO/NCD/NCS/99.2)) ein metabolisches Syndrom auf, wenn sie neben dem Nachweis der Insulinresistenz zwei der folgenden Kriterien erfüllt:
- Hypertonie definiert als antihypertensive Therapie und/oder Blutdruck > 160/90 mmHg,
- Dyslipidämie definiert als erhöhte Plasmatriglyzeride (≥ 1,7 mmol/l) und/oder erniedrigtes HDL-Cholesterol (< 0,9 mmol bei Männern bzw. < 1,0 mmol bei Frauen),
- Adipositas BMI (30 kg/m²) und/oder einen Taillen-/Hüftumfangsquotienten > 0,85 bei Frauen und > 0,9 bei Männern,
- Mikroalbuminurie (Albuminexkretionsrate (20 µg/M).
   Weist eine Person Typ II-Diabetes oder eine verminderte Glucosetoleranz auf, dann hat sie ein metabolisches Syndrom, wenn zwei der oben genannten Kriterien erfüllt sind.

Besonders bevorzugt ist die erfindungsgemäße Anwendung zur Vorbeugung und/oder Behandlung von Patienten mit Insulinresistenz, Arteriosklerose, Lipid-, Eiweiß- und/oder Kohlehydratstoffwechselstörungen. Überraschend hat sich bei der regelmäßigen erfindungsgemäßen Anwendung herausgestellt, dass vor allem bei prädisponierten Personen die Insulin-Empfindlichkeit deutlich und dauerhaft verbessert werden kann, wobei sowohl der Blutzucker- als auch der Insulin-Spiegel auf einem äußerst niedrigem Niveau gehalten werden. Dabei hat sich die Getreideschlempe als nahezu Insulin-neutral gezeigt, sodass die Insulin-Hunger-Spirale nur in äußerst geringem Maß in Bewegung gesetzt wurde.

Hinzu kam die ebenfalls nicht vorherzusehende Beobachtung, dass die Verbesserung der im Zusammenhang mit Diabetes als kritisch anzusehenden Faktoren des metablischen Syndroms, vor allem auch ohne eine sonst übliche scharfe Reduktionsdiät erzielt werden kann, was vermutlich dem äußerst niedrigem glykämischen Index (GI) der verwendeten Getreideschlempe zuzurechnen ist. Der glykämische Index klassifiziert kohlenhydrathaltige Lebensmittel nach ihrer Blutzucker steigernden Wirkung. Er gibt in Zahlen die Blutzucker steigernde Wirkung der Kohlenhydrate bzw. kohlenhydrathaltigen Lebensmittel an. Die Blutzucker steigernde Wirkung von Glukose dient als Referenz und hat den Referenzwert von 100 zugewiesen bekommen. Der GI wird definiert als die Fläche unter der Kurve der Blutzuckerwerte, d.h. ein GI von 50 bedeutet, dass der Blutzuckeranstieg dieses Produkts nur die Hälfte des Anstiegs der Blutglukose über den Nüchternwert hinaus ausmacht. Ein hoher glykämischer Index wird zwischen 70 und 100, ein mittlerer zwischen 55 und 70, und ein niedriger unter 55 definiert. Außerdem konnte eine Senkung des Fruktosamin, des Blutdrucks sowie der Blutfette Triglyceride und Cholesterin erreicht werden.

Die Verwendung gemäß vorliegender Erfindung basiert insbesondere auf dem Einsatz einer speziell hergestellten Getreideschlempe, wie sie in WO 01/10245 beschrieben ist. Es wird deshalb bevorzugt vorgeschlagen, dass erfindungsgemäß eine Getreideschlempe eingesetzt wird, die hergestellt wurde, indem die Getreideschlempe direkt aus dem Brennkessel entnommen, dann eingedickt, aber nicht getrocknet, und anschließend mit Milch und einer Joghurt- und/oder Butterei-Kultur fermentiert wird.

Als besonders geeignet hat sich dabei der Einsatz einer Getreideschlempe gezeigt, die hergestellt wurde, indem
a) eine rohe und mit Hefekulturen meist im Rahmen der alkoholischen Gärung vorfermentierte Getreideschlempe bei einem Druck von 100 bis 800 mbar auf ca. 15 bis 17 % TM (Trockenmasse) eingedickt wird, dann
b) mit einer bereits mit der Joghurt- und/oder Butterei-Kultur angeimpften Milch vermischt wird,
c) dieses Gemisch entweder mit einer Joghurt-Kultur bei einer Temperatur von 38 bis 48 °C 10 bis 20 Stunden fermentiert oder dieses Gemisch mit einer Butterei-Kultur bei einer Temperatur von 18 bis 24 °C bis zu 36 Stunden fermentiert wird, und danach
d) mit weiterer Milch oder Milchkonzentrat vermischt auf Temperaturen unter 15 °C gekühlt wird, und
e) die ggf. abschließend sprühgetrocknet wird.

Man erhält auf diese Weise ein ballaststoffreiches, eiweißhaltiges Vollkornerzeugnis, dessen Kulturen z.B. in einer nicht-erhitzten, aber sterilen Flüssigformulierung probiotische Eigenschaften entfalten. Darüber hinaus enthält die so mehrfach fermentierte Getreideschlempe essentielle Fettsäuren, lebenswichtige Vitamine, Mineralstoffe und Spurenelemente. Durch die nachgeschaltete Sprühtrocknung verlieren die verwendeten Kulturen ihre Lebensfähigkeit, was das Produkt äußerst stabil und lagerfähig macht. Auch ein sich möglicherweise während der Lagerung erhöhender Feuchtigkeitsgehalt kann so die Fermentation nicht wieder einsetzen lassen.

Die so erhaltenen Produkte können selbstverständlich mit weiteren und insbesondere fermentierbaren Zusätzen versehen werden, die die Eigenschaften des Endprodukts zusätzlich positiv beeinflussen. So wurde herausgefunden, dass bspw. Honig, Lecithine und/oder Sojaeiweiß mit Getreideschlempe Komplexverbindungen eingehen können. Durch chemisch/physikalische Wechselwirkungen zwischen den Lecithinen, dem Eiweiß und den einzelnen Stoffgruppen der Getreideschlempe entstehen so Nährstoffsysteme, die sich hinsichtlich ihrer chemischen, physikalischen und biologischen Verhaltensweisen erheblich von denen der Einzelkomponenten unterscheiden. Selbstverständlich ist auch das bloße Abmischen sowohl des sprühgetrockneten, als auch des noch nicht sprühgetrockneten Produktes mit Mineralstoffen, Vitaminen, Geschmacksstoffen, Aromastoffen und Farbstoffen möglich, was die Produktvielfalt zusätzlich erhöht.

Hervorzuheben ist, dass all den möglichen Produkten auf Getreideschlempe-Basis eine im Hinblick auf den Geschmack und den Geruch spezifische Qualität zu eigen ist, die nicht mehr an die typische rohe Getreideschlempe erinnert und die deshalb von den Verbrauchern sehr gut akzeptiert wird, was sich insbesondere in einer erhöhten Kompliance äußert.

Prinzipiell ist die Verwendung im beanspruchten Rahmen auf kein spezielles Ausgangsmaterial beschränkt, jedoch wird eine Getreideschlempe auf Weizen-, Gerste-, Roggen-, Hafer-, Mais- und/oder Reis-Basis empfohlen.

Für das Erreichen der angestrebten Wirkungen sollte die verwendete Getreideschlempe im Rahmen der vorliegenden Erfindung in einer Tagesdosis von 100 bis 400 g, bevorzugt 150 bis 300 g und besonders bevorzugt 200 bis 250 g, verabreicht werden, wobei 2 bis 5 Einzeldosen pro Tag, und insbesondere gleichgroße Einzeldosen besonders zu empfehlen sind.

Als ideale Verabreichungsform haben sich für die Getreideschlempe solche in viskoser Form gezeigt, wobei sie besonders bevorzugt in Wasser oder fermentierter Milch und ganz besonders bevorzugt in Sauermilch, Buttermilch, Joghurt, Quark oder Kefir eingerührt verabreicht wird.

Je nach Wunsch kann die Zubereitung auch mit Süßstoffen, wie z. B. Fructose (Zuckeraustauschstoff), Cyclamat oder Saccharin abgeschmeckt werden. Nicht enthalten sollte sie jedoch Haushaltszucker (Saccharose), Traubenzucker (Glukose) und Malzzucker (Maltose). Die Getreideschlempe kann zwar auch in Früchtetees oder Fruchtsäfte oder diese enthaltende Getränke eingerührt werden, jedoch enthalten Fruchtsäfte im Gegensatz zu Früchtetees zumeist Zucker, die über einen Insulin-Anstieg die Fettverbrennung beeinträchtigen würden. Insgesamt ist darauf zu achten, dass die verwendeten Einrührmedien weder zuviel Zucker, noch zuviel Fett enthalten.

Die vorliegende Erfindung sieht ebenfalls vor, die Getreideschlempe als Mahlzeitersatz und/oder als Mahlzeitergänzung zu verabreichen, was besonders bevorzugt über einen Zeitraum von mindestens 3 aufeinander folgenden Wochen erfolgen sollte. Falls das Wohlbefinden es zulässt, kann die erfindungsgemäße Verwendung auch besonders bevorzugt zeitlich uneingeschränkt auf Dauer vorgenommen werden.

Falls gewünscht oder medizinisch empfohlen, kann die erfindungsgemäße Verwendung auch in Kombination und/oder gemeinsam mit weiteren anti-diabetisch wirkenden oder dem Diabetes vorbeugenden Wirkstoffen, bevorzugt dem Diabetes Typ II entgegenwirkenden oder vorbeugenden Wirkstoffen erfolgen, was das gewünschte Resultat zusätzlich verstärkt.

Besonders bevorzugt ist die vorgeschlagene Verwendung in Form von und/oder in Kombination mit und/oder gemeinsam mit Nahrungsergänzungsmitteln, Funktionsnahrungsmitteln (Functional Food) und/oder Spezialernährung.

Nahrungsergänzungsmitteln sind Lebensmittel, die wegen ihres Nährwertes verzehrt werden, um die tägliche gewöhnliche Nahrung gesunder Personen zu ergänzen, deren Zufuhr an einem oder mehreren Nährstoffen aus dieser gewöhnlichen Nahrung marginal, zweifelhaft oder unzureichend ist. Ziel ist eine ausreichende Versorgung des Körpers mit Nährstoffen oder anderen ernährungsphysiologisch wirksamen Stoffen wie Vitaminen, Mineralstoffen einschl. Spurenelementen, essentiellen Fettsäuren, Aminosäuren, Eiweißstoffe und/oder Kohlenhydraten.
Als "Functional Food", funktionelle Lebensmittel oder Funktionsnahrungsmittel werden Lebensmittel definiert, die einen spezifischen Zusatznutzen, der über den ernährungsphysiologischen Nutzen der darin enthaltenen Nährstoffe hinaus geht, enthalten. Dieser Zusatznutzen wird durch Zugabe bestimmter Substanzen oder durch Veränderung der ursprünglichen Eigenschaften, beispielsweise durch Gentechnik, erreicht. Die am häufgisten zugesetzten Substanzen sind dabei Vitamine, Spurenelemente, Pro- und Präbiotika, bestimmte Fettsäuren oder Fettersatzstoffe, sekundäre Pflanzeninhaltsstoffe oder Enzyme.
Zur Spezialnahrung gehören zum Beispiel Sondennahrung, sterile Kost, milchfreie Spezialnahrung für Säuglinge, Laktose-allergenarme Spezialnahrung zur Ernährung von Säuglingen, etc. Durch diese vorgeschlagene Verwendung in Form von und/oder in Kombination mit Nahrungsergänzungsmitteln, Funktionsnahrungsmitteln und/oder Spezialernährung kann die Kompliance bei den Patienten zusätzlich eine deutliche Steigerung erfahren und die vorteilhafte Wirkung der Getreideschlempe durch andere Wirkstoffe verstärkt und/oder ergänzt werden.

Dies kommt insbesondere dann zum Tragen, wenn die Getreideschlempe in Form von und/oder in Kombination mit und/oder gemeinsam mit Kreatin, Kreatin-Monohydrat, α-Liponsäure, Phytosterol, mehrfach ungesättigten Fettsäuren und/oder Phospholipiden sowie deren Derivate und/oder Gemisch eingesetzt wird. Besonders bevorzugt ist die Verwendung von mehrfach ungesättigten Fettsäuren wie z.B. Dokosahexaensäure (DHA), Eikosapentaensäure (EPA) und/oder konjugierte Linolsäure (CLA), sowie von Phospholipiden wie z.B. Lecithin oder Phosphatidylserin.

Im Rahmen der erfindungsgemäßen Verwendung einer speziellen mehrfach fermentierten Getreideschlempe kann der Organismus mit allen wesentlichen Makro- und Mikronährstoffen versorgt werden. Die in der Getreideschlempe enthaltenen Proteine versorgen und schonen das Muskelgewebe und es werden aufgrund der spezifischen Zusammensetzung der Getreideschlempe und deren Verwendung als Mahlzeitersatz vorwiegend Fette verbraucht, die zudem in Zusammenspiel mit den in der Getreideschlempe enthaltenen Komponenten optimal verbrannt werden.

Ein zusätzlicher Aspekt ist darin zu sehen, dass die Verwendung der mehrfach fermentierten Getreideschlempe einen regulierenden Einfluss auf die Darmflora ausübt. Besonders bevorzugt zeigt die Verwendung der spezifischen, löslichen und unlöslichen Ballaststoffe der Getreideschlempe einen spürbar positiven und regelnden Einfluss auf die Darmflora, was insbesondere bei der Verwendung der Getreideschlempe als Mahlzeitersatz und/oder als Mahlzeitergänzung bei ausreichender Aufnahme von Flüssigkeit positiv zum Tragen kommt.

Die nachfolgenden Beispiele verdeutlichen die Vorteile einer speziellen, mehrfach fermentierten Getreideschlempe bei der erfindungsgemäßen Verwendung.

### Beispiele

Es wurde eine kontrollierte, offene, randomisierte Cross-Over-Studie durchgeführt, bei der die Insulin-Sensitivität bei Probanden mit Adipositas und gleichzeitig erhöhtem Nüchtern-Blutzucker (Prädiabetiker) untersucht wurde. Eingesetzt wurde eine gemäß WO 01/10 245 hergestellte, doppelt-fermentierte Getreideschlempe mit einem hohen Anteil an Stärke-reduziertem Weizenvollkorn, welche unter der Marke "Vibamin^{®}" vertrieben wird.

Die Studiendauer betrug für jeden Teilnehmer ca. 10 Wochen, wobei die Probanden im cross-over Verfahren jeweils 4 Wochen lang mit "Vibamin^{®}" bzw. mit einem käuflich zu erwerbenden Vergleichsprodukt gemäß erarbeitetem Behandlungsplan behandelt wurden; zwischen den einzelnen Behandlungszeiträumen waren 2 Wochen für die sogenannte "Wash-Out"-Phase vorgesehen. Als Vergleich zur Verwendung von "Vibamin^{®}" wurde ein den Marktführern zugerechnetes Produkt eingesetzt, welches als Trinkmahlzeit zur Gewichtsreduzierung im freien Handel erworben werden kann.

Insgesamt waren an dieser offenen, randomisierten, zweifach "Cross-Over-Studie" 30 Teilnehmer im Alter zwischen 18 und 70 Jahren beteiligt, deren Body-Mass-Index (BMI) > 29 und < 40 kg/m² betrug. Die Nüchtern-Blutzucker-Werte lagen zu Beginn der Studie zwischen 110 und 126 mg/dl. Eine anti-diabetische Zusatzbehandlung mit Tabletten oder Insulin fand nicht statt. Außerdem wurden bei den Probanden keine schwerwiegenden Vorerkrankungen festgestellt.

Zwei tägliche (Haupt-)Mahlzeiten wurden durch Zufuhr von mindestens je 45 g "Vibamin^{®}" bzw. dem Vergleichsprodukt dargestellt, die übrige Hauptmahlzeit wurde wie sonst üblich eingenommen. Den Probanden war es freigestellt, aus "Vibamin^{®}" oder dem Vergleichsprodukt bestehende Zwischenmahlzeiten zu beliebigen Tageszeiten zu verzehren.

Es war allerdings zu beachten, dass bei den Probanden der Studie unter "Vibamin^{®}" oder dem Vergleichsprodukt eine geringe Reduzierung der täglichen Kalorien-Zufuhr (Richtwert der ausgewogenen Ernährung ca. 1700 kcal/Tag) erfolgte.

Zu Beginn der Studie wurde eine ausführliche Diätberatung durchgeführt, bei der den Teilnehmern das diätetische Konzept erläutert wurde.

Nach einer Woche Diät sowohl mit "Vibamin^{®}" als auch mit dem Vergleichsprodukt erfolgte ein Interims-Besuch, bei dem die Teilnehmer nach ihren persönlichen Erfahrungen mit der Diät befragt und das aktuelle Körpergewicht ermittelt wurde. Ergänzend wurden die selben Parameter erhoben wie bei den Besuchen zu Beginn und zum Ende der Behandlung.

Von den Probanden wurde während der Studie ein standardisiertes Tagebuch geführt, in dem Tageszeit und Menge von verzehrtem "Vibamin^{®}" bzw. Vergleichsprodukt protokolliert wurden.

Durch unangekündigte Anrufe bei den Probanden der Studie wurde die Kompliance der Teilnehmer erhöht.

Im Vordergrund der Studie stand die Änderung des Insulin-Resistenz-Index HOMA-IR (HOMA-Index) nach je 4 Wochen Diät mit "Vibamin^{®}" bzw. dem Vergleichsprodukt bei Prädiabetikern (Probanden mit Adipositas und gleichzeitig erhöhtem Nüchtern-Blutzucker). Hierbei war die absolute Verbesserung des HOMA-Index von Interesse, mehr noch aber die relative Verbesserung des HOMA-Index nach Herausrechnen des durch den Gewichtsverlust hervorgerufenen Effektes. Auf diese Weise kann der ausschließlich durch die Behandlung spezifisch hervorgerufene Effekt ermittelt werden.

Festgestellt wurde ebenfalls die Änderung der Körpergewichts nach je 4 Wochen Diät, die Änderung von Fructosamingehalt, Nüchtern-Blutzucker, Nüchterninsulin, Waist-to-hip ratio, Triglyceriden und Cholesterin sowie des Blutdrucks. Ebenfalls erhoben wurden das subjektive Wohlbefinden und das postprandiale Sättigungsgefühl nach je 4 Wochen mit Hilfe eines standardisierten Fragebogens.

Beim nachfolgenden Ergebnis dieser Studie ist insbesondere die signifikante Senkung des Nüchtern-Blutzucker-Spiegels bei sämtlichen Probanden hervorzuheben. Ebenfalls ausnahmslos konnte bei allen behandelten Probanden eine drastische und kontinuierlich erfolgende Gewichtsreduzierung (u.a. erfasst als BMI) festgestellt werden. Herauszustellen ist aber für die "Vibamin^{®}" behandelten Probanden, dass die Verbesserung des Nüchternblutzuckers, des Nüchterninsulin und des Hauptparameters HOMA-Index, auch nach dem Herausrechnen der Störgröße "Gewicht", weiterhin signifikant bleibt (p=0,005; p=0,03 und p=0,007), während beim Vergleichsprodukt nur die Gewichtsreduktion die Verbesserung hervorruft.

Negative Auswirkungen auf sonstige Vitalzeichen, wie Blutdruck, Puls oder EKG, wurden weder unter "Vibamin^{®}" noch unter dem Vergleichsprodukt festgestellt.

Drei der Probanden beendeten auf eigenen Wunsch die Studie vorzeitig.

**Tabelle 1: HOMA-IR (mit p-Values absolut)**

| Vergleichsprodukt | | | | Vibamin | | | |
|---|---|---|---|---|---|---|---|
| Proband | Vorher | Nacher | Differenz | Proband | Vorher | Nacher | Differenz |
| 301 | 3,3 | 3,39 | -0,09 | 301 | 4,82 | 3,53 | 1,3 |
| 302 | 3,85 | 3,06 | 0,79 | 302 | 3,92 | 1,14 | 2,78 |
| 303 | 3,22 | 2,44 | 0,77 | 303 | 5,77 | 2,84 | 2,94 |
| 304 | 3,44 | 1,79 | 1,65 | 304 | 5,04 | 2,37 | 2,67 |
| 305 | 1,94 | 1,64 | 0,3 | 305 | 1,79 | 1,89 | -0,1 |
| 306 | 9,1 | 9,91 | -0,82 | 306 | 10,5 | 10,1 | 0,47 |
| 307 | 7,25 | 11,1 | -3,86 | 307 | 6,57 | 8,87 | -2,31 |
| 308 | 2,57 | 5,25 | -2,68 | 308 | 4,89 | 3,78 | 1,12 |
| 309 | 6,14 | 3,49 | 2,65 | 309 | 3,54 | 2,83 | 0,72 |
| 310 | 3,62 | 3,52 | 0,1 | 310 | 5,11 | 2,75 | 2,36 |
| 311 | 2,94 | 2,84 | 0,11 | 311 | 1,91 | 2,73 | -0,83 |
| 312 | 5,22 | 4,21 | 1,01 | 312 | 4,06 | 3,73 | 0,33 |
| 313 | 0,7 | 1,3 | -0,6 | 313 | 1,93 | 1,41 | 0,52 |
| 314 | 2,7 | 1,56 | 1,14 | 314 | 2,37 | 2,51 | -0,14 |
| 316 | 5,56 | 4 | 1,56 | 316 | 4,55 | 3,66 | 0,89 |
| 317 | 4,13 | 3,55 | 0,58 | 317 | 2,85 | 3,54 | -0,69 |
| 318 | 2,24 | 1,94 | 0,29 | 318 | 2,75 | 1,97 | 0,79 |
| 320 | 2,78 | 1,72 | 1,07 | 320 | 2,57 | 1,39 | 1,18 |
| 321 | 7,35 | 5,52 | 1,83 | 321 | 3,35 | 3,56 | -0,21 |
| 322 | 2,44 | 1,17 | 1,27 | 322 | 2,04 | 0,93 | 1,1 |
| 323 | 4,11 | 3,35 | 0,76 | 323 | 3,32 | 3,47 | -0,15 |
| 324 | 6,43 | 5,92 | 0,51 | 324 | 3,9 | 2,8 | 1,11 |
| 325 | 8,87 | 3,86 | 5,01 | 325 | 3,53 | 4,61 | -1,08 |
| 326 | 9,63 | 6,7 | 2,93 | 326 | 6,42 | 5,17 | 1,25 |
| 328 | 3,63 | 1,77 | 1,85 | 328 | 2,51 | 1,99 | 0,51 |
| 330 | 5,36 | 3,84 | 1,52 | 330 | 3,67 | 3,28 | 0,4 |
| 331 | 4,31 | 1,63 | 2,69 | 331 | 5,82 | 3,77 | 2,04 |
| 333 | 5,36 | 5,39 | -0,03 | 333 | 5,91 | 4,18 | 1,72 |
| 334 | 13,31 | 3,55 | 9,76 | 334 | 1,7 | 2,37 | -0,68 |
| 335 | 3,62 | 2,38 | 1,24 | 335 | 4,16 | 2,37 | 1,79 |
| 336 | 3,41 | 2,38 | 1,03 | 336 | 4,07 | 2,87 | 1,2 |
| | | | | | | | |
| Mean | 4,8 | 3,7 | 1,1 | Mean | 4,0 | 3,3 | 0,7 |
| STABW | 2,7 | 2,3 | 2,3 | STABW | 1,9 | 1,9 | 1,2 |
| t-test | | | 0,010 | t-test | | | 0,002 |

**Tabelle 2: Nüchterninsulin (mit p-Values absolut)**

| Vergleichsprodukt | | | | Vibamin | | | |
|---|---|---|---|---|---|---|---|
| Proband | Vorher | Nacher | Differenz | Proband | Vorher | Nacher | Differenz |
| 301 | 10,8 | 11,9 | -1,1 | 301 | 17,0 | 13,3 | 3,7 |
| 302 | 14,6 | 12,8 | 1,8 | 302 | 15,9 | 5,0 | 10,9 |
| 303 | 11,8 | 10,0 | 1,8 | 303 | 18,5 | 10,7 | 7,8 |
| 304 | 12,4 | 7,8 | 4,6 | 304 | 18,4 | 9,4 | 9,0 |
| 305 | 6,5 | 6,1 | 0,4 | 305 | 6,6 | 7,7 | -1,1 |
| 306 | 29,5 | 28,5 | 1,0 | 306 | 32,7 | 28,8 | 3,9 |
| 307 | 24,7 | 39,5 | -14,8 | 307 | 24,2 | 32,4 | -8,2 |
| 308 | 9,6 | 15,2 | -5,6 | 308 | 17,1 | 17,9 | -0,8 |
| 309 | 23,7 | 15,8 | 7,9 | 309 | 14,8 | 12,8 | 2,0 |
| 310 | 11,2 | 11,2 | 0,0 | 310 | 15,7 | 9,5 | 6,2 |
| 311 | 10,9 | 11,0 | -0,1 | 311 | 6,9 | 10,5 | -3,6 |
| 312 | 14,6 | 12,5 | 2,1 | 312 | 13,0 | 12,0 | 1,0 |
| 313 | 3,4 | 6,1 | -2,7 | 313 | 7,9 | 5,6 | 2,3 |
| 314 | 12,8 | 7,4 | 5,4 | 314 | 10,8 | 12,1 | -1,3 |
| 316 | 16,2 | 12,0 | 4,2 | 316 | 14,2 | 1 1,0 | 3,2 |
| 317 | 12,5 | 11,6 | 0,9 | 317 | 9,5 | 12,2 | -2,7 |
| 318 | 9,3 | 7,8 | 1,5 | 318 | 9,5 | 7,6 | 1,9 |
| 320 | 12,2 | 8,1 | 4,1 | 320 | 11,4 | 6,7 | 4,7 |
| 321 | 20,4 | 18,8 | 1,6 | 321 | 12,3 | 13,0 | -0,7 |
| 322 | 9,0 | 4,6 | 4,4 | 322 | 7,5 | 4,1 | 3,4 |
| 323 | 11,1 | 9,4 | 1,7 | 323 | 8,6 | 10,0 | -1,4 |
| 324 | 21,1 | 19,9 | 1,2 | 324 | 13,3 | 10,5 | 2,8 |
| 325 | 30,1 | 17,1 | 13,0 | 325 | 13,5 | 20,0 | -6,5 |
| 326 | 23,3 | 19,6 | 3,7 | 326 | 19,7 | 16,5 | 3,2 |
| 328 | 10,5 | 6,3 | 4,2 | 328 | 8,5 | 7,7 | 0,8 |
| 330 | 16,1 | 15,8 | 0,3 | 330 | 13,3 | 12,3 | 1,0 |
| 331 | 15,9 | 7,3 | 8,6 | 331 | 20,5 | 15,6 | 4,9 |
| 333 | 20,6 | 21,2 | -0,6 | 333 | 21,1 | 16,0 | 5,1 |
| 334 | 43,7 | 14,4 | 29,3 | 334 | 7,9 | 10,4 | -2,5 |
| 335 | 14,3 | 8,6 | 5,7 | 335 | 15,0 | 10,4 | 4,6 |
| 336 | 12,3 | 9,5 | 2,8 | 336 | 15,7 | 11,4 | 4,3 |
| | | | | | | | |
| Mean | 16,0 | 13,2 | 2,8 | Mean | 14,2 | 12,4 | 1,9 |
| STABW | 8,2 | 7,3 | 6,8 | STABW | 5,8 | 6,1 | 4,2 |
| t-test | | | 0,028 | t-test | | | 0,019 |

**Tabelle 3: Nüchternglucose (mit p-Values absolut)**

| Vergleichsprodukt | | | | Vibamin | | | |
|---|---|---|---|---|---|---|---|
| Proband | Vorher | Nacher | Differenz | Proband | Vorher | Nacher | Differenz |
| 301 | 124 | 116 | 8,5 | 301 | 115 | 108 | 7,5 |
| 302 | 107 | 97 | 10 | 302 | 100 | 92,5 | 7,5 |
| 303 | 110,5 | 99 | 11,5 | 303 | 127 | 108 | 19 |
| 304 | 112,5 | 93 | 19,5 | 304 | 111 | 102 | 9 |
| 305 | 121 | 109 | 12 | 305 | 110 | 99,5 | 10,5 |
| 306 | 125 | 141 | -16 | 306 | 131 | 142 | -11 |
| 307 | 119 | 114 | 5 | 307 | 110 | 111 | -1 |
| 308 | 108,5 | 140 | -31,5 | 308 | 116 | 85,5 | 30,5 |
| 309 | 105 | 89,5 | 15,5 | 309 | 97 | 89,5 | 7,5 |
| 310 | 131 | 128 | 3,5 | 310 | 132 | 118 | 14,5 |
| 311 | 109,5 | 105 | 5 | 311 | 112 | 106 | 6,5 |
| 312 | 145 | 137 | 8,5 | 312 | 127 | 126 | 0,5 |
| 313 | 84 | 86,5 | -2,5 | 313 | 99 | 102 | -3 |
| 314 | 85,5 | 85,5 | 0 | 314 | 89 | 84 | 5 |
| 316 | 139 | 135 | 4 | 316 | 130 | 135 | -5 |
| 317 | 134 | 124 | 10 | 317 | 122 | 118 | 4 |
| 318 | 97,5 | 101 | -3,5 | 318 | 118 | 105 | 12,5 |
| 320 | 92,5 | 86 | 6,5 | 320 | 91,5 | 84 | 7,5 |
| 321 | 146 | 119 | 27 | 321 | 111 | 111 | -0,5 |
| 322 | 110 | 103 | 7 | 322 | 110 | 92 | 18 |
| 323 | 150 | 145 | 5,5 | 323 | 157 | 141 | 16 |
| 324 | 123,5 | 121 | 3 | 324 | 119 | 108 | 11 |
| 325 | 119,5 | 91,5 | 28 | 325 | 106 | 93,5 | 12,5 |
| 326 | 167,5 | 139 | 29 | 326 | 132 | 127 | 5 |
| 328 | 140 | 114 | 26 | 328 | 120 | 105 | 14,5 |
| 330 | 135 | 98,5 | 36,5 | 330 | 112 | 108 | 4 |
| 331 | 110 | 90,5 | 19,5 | 331 | 115 | 98 | 17 |
| 333 | 105,5 | 103 | 2,5 | 333 | 114 | 106 | 7,5 |
| 334 | 123,5 | 100 | 23,5 | 334 | 87 | 92,5 | -5,5 |
| 335 | 102,5 | 112 | -9,5 | 335 | 113 | 92,5 | 20 |
| 336 | 112,5 | 102 | 11 | 336 | 105 | 102 | 3 |
| | | | | | | | |
| Mean | 119 | 110 | 9 | Mean | 114 | 106 | 8 |
| STABW | 19 | 18 | 14 | STABW | 14 | 15 | 9 |
| t-test | | | 0,001 | t-test | | | 0,000021 |

**Tabelle 4: Gewicht (mit p-Values absolut)**

| Vergleichsprodukt | | | | Vibamin | | | |
|---|---|---|---|---|---|---|---|
| Proband | Vorher | Nacher | Differenz | Proband | Vorher | Nacher | Differenz |
| 301 | 106,6 | 103 | 3,4 | 301 | 106 | 104 | 1,2 |
| 302 | 92 | 87,1 | 4,9 | 302 | 85,4 | 82,7 | 2,7 |
| 303 | 98,7 | 96,5 | 2,2 | 303 | 99,9 | 97,9 | 2 |
| 304 | 93,9 | 90,6 | 3,3 | 304 | 93,4 | 92,4 | 1 |
| 305 | 94 | 89,5 | 4,5 | 305 | 90,5 | 90,5 | 0 |
| 306 | 130,4 | 127 | 3,7 | 306 | 128 | 128 | 0,1 |
| 307 | 106,6 | 103 | 3,2 | 307 | 109 | 106 | 2,8 |
| 308 | 116,4 | 113 | 3,3 | 308 | 115 | 114 | 1,3 |
| 309 | 107,7 | 104 | 3,3 | 309 | 106 | 108 | -2,6 |
| 310 | 97,6 | 95,3 | 2,3 | 310 | 95,7 | 93,7 | 2 |
| 311 | 90,6 | 91 | -0,4 | 311 | 92,6 | 89,7 | 2,9 |
| 312 | 80,2 | 79,4 | 0,8 | 312 | 79,8 | 78 | 1,8 |
| 313 | 108 | 106 | 2,4 | 313 | 114 | 107 | 7,5 |
| 314 | 107,7 | 105 | 2,9 | 314 | 111 | 106 | 4,9 |
| 316 | 84,5 | 80,5 | 4 | 316 | 80 | 77 | 3 |
| 317 | 96,8 | 93,7 | 3,1 | 317 | 92,8 | 90,8 | 2 |
| 318 | 85,3 | 80,8 | 4,5 | 318 | 91,3 | 85,7 | 5,6 |
| 320 | 88,4 | 82,1 | 6,3 | 320 | 81,8 | 78,9 | 2,9 |
| 321 | 95,8 | 91,4 | 4,4 | 321 | 91,1 | 87,8 | 3,3 |
| 322 | 96,5 | 91,1 | 5,4 | 322 | 92,9 | 88,8 | 4,1 |
| 323 | 108,7 | 105 | 3,5 | 323 | 106 | 102 | 4 |
| 324 | 109 | 104 | 4,8 | 324 | 106 | 105 | 1,3 |
| 325 | 110,8 | 106 | 5,2 | 325 | 108 | 104 | 4,4 |
| 326 | 125 | 123 | 1,7 | 326 | 123 | 122 | 0,8 |
| 328 | 78,2 | 75,3 | 2,9 | 328 | 75,5 | 72,6 | 2,9 |
| 330 | 99,9 | 96 | 3,9 | 330 | 95,6 | 92,4 | 3,2 |
| 331 | 93,6 | 91,9 | 1,7 | 331 | 95,7 | 92,6 | 3,1 |
| 333 | 98,9 | 98,2 | 0,7 | 333 | 98,2 | 99,9 | -1,7 |
| 334 | 85,5 | 80,5 | 5 | 334 | 79,5 | 77 | 2,5 |
| 335 | 69,8 | 68,4 | 1,4 | 335 | 72,7 | 68,4 | 4,3 |
| 336 | 105,1 | 105 | 0,6 | 336 | 109 | 105 | 4,2 |
| | | | | | | | |
| Mean | 98,8 | 95,6 | 3,2 | Mean | 97,5 | 95,0 | 2,5 |
| STABW | 13,2 | 13,2 | 1,6 | STABW | 13,7 | 14,1 | 2,0 |
| t-test | | | 0,00000000 | t-test | | | 0,0000001333 |

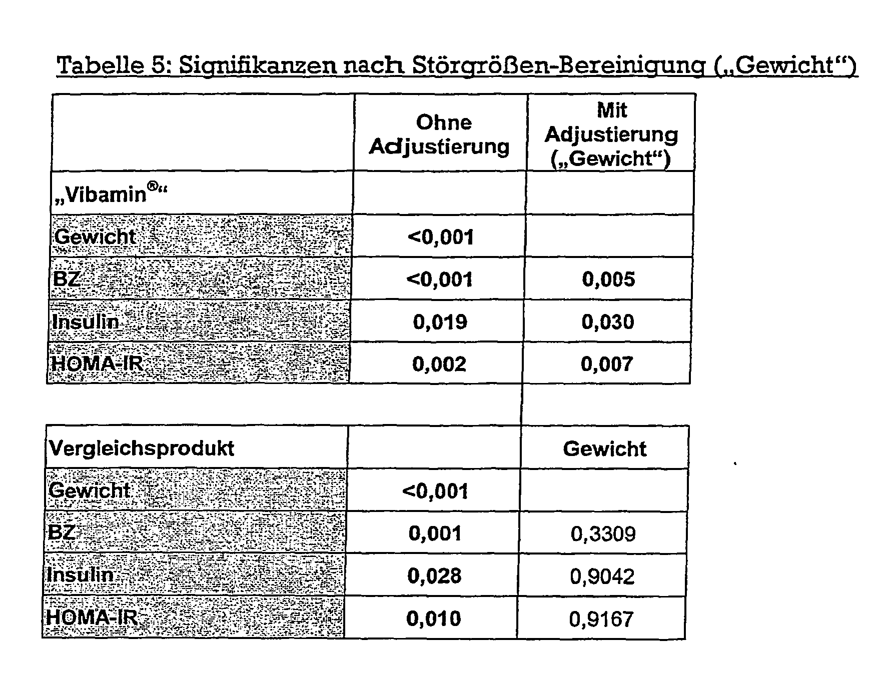

## Patentansprüche

1. Verwendung einer Getreideschlempe, die zusätzlich zu einer Fermentierung mit Hefe auch mit einer Joghurt- und/oder Butterei-Kultur fermentiert wurde, zur Herstellung einer Zusammensetzung zur Vorbeugung und/oder Behandlung erhöhter Blutzucker-Werte.

2. Verwendung nach Anspruch 1, wobei die erhöhten Blutzuckerwerte ausgewählt sind aus Nüchtern- und/oder postprandialen Blutzuckerwerten.

3. Verwendung nach Anspruch 1, wobei eine Getreideschlempe eingesetzt wird, deren Reststärkegehalt maximal 4 Gewichts-%, bezogen auf das Gesamtgewicht der Getreideschlempe, beträgt.

4. Verwendung nach einem der Ansprüche 1 bis 3 zur Herstellung einer Zusammensetzung zur Vorbeugung und/oder Behandlung von Patienten mit Prädiabetes und/oder Diabetes und/oder einer Prädisposition dafür und damit zusammenhängender Formen und/oder Vorformen.

5. Verwendung nach Anspruch 4, wobei Diabetes ausgewählt ist aus Diabetes Typ Ia und Ib, Typ IIa und IIb und/oder autosomal vererbtem Diabetes des Jugendalters, und wobei die damit zusammenhängenden Formen und/oder Vorformen ausgewählt sind aus sekundären Diabetes-Formen, potentiellem Diabetes, latentem Diabetes, verminderter Glucosetoleranz und/oder klinisch manifestem Diabetes.

6. Verwendung nach Anspruch 5, wobei es sich bei Diabetes um Diabetes Typ II handelt.

7. Verwendung nach einem der Ansprüche 1 bis 6 weiterhin zur Herstellung einer Zusammensetzung zur Reduzierung des Körpergewichts.

8. Verwendung nach einem der Ansprüche 1 bis 7 zur Herstellung einer Zusammensetzung zur Vorbeugung und/oder Behandlung von Patienten mit Adipositas.

9. Verwendung nach Anspruch 8 zur Herstellung einer Zusammensetzung zur Vorbeugung und/oder Behandlung von Patienten mit Adipositas und gleichzeitig erhöhten Nüchtern-Blutzuckerwerten.

10. Verwendung nach einem der Ansprüche 1 bis 9 weiterhin zur Herstellung einer Zusammensetzung zur Vorbeugung und/oder Behandlung von Patienten mit metabolischem Syndrom.

11. Verwendung nach Anspruch 10 zur Herstellung einer Zusammensetzung zur Vorbeugung und/oder Behandlung von Patienten mit Insulinresistenz, Arteriosklerose, Lipid-, Eiweiß- und/oder Kohlehydratstoffwechselstörungen.

12. Verwendung nach einem der Ansprüche 1 bis 11, wobei die Getreideschlempe einen niedrigen glykämischen Index aufweist.

13. Verwendung nach einem der Ansprüche 1 bis 12, wobei eine Getreideschlempe eingesetzt wird, die hergestellt wurde, indem die Getreideschlempe direkt aus dem Brennkessel entnommen, dann eingedickt, aber nicht getrocknet, und anschließend mit Milch und einer Joghurt- und/oder Butterei-Kultur fermentiert wird.

14. Verwendung nach Anspruch 13, wobei eine Getreideschlempe eingesetzt wird, die hergestellt wurde, indem
a) die Getreideschlempe bei einem Druck von 100 bis 800 mbar auf ca. 15 bis 17 % Trockenmasse eingedickt wird, dann
b) mit einer bereits mit der Joghurt- und/oder Butterei-Kultur angeimpften Milch vermischt wird,
c) dieses Gemisch entweder mit einer Joghurt-Kultur bei einer Temperatur von 38 bis 48 °C 10 bis 20 Stunden fermentiert oder dieses Gemisch mit einer Butterei-Kultur bei einer Temperatur von 18 bis 24 °C bis zu 36 Stunden fermentiert wird, und danach
d) mit weiterer Milch oder Milchkonzentrat vermischt auf Temperaturen unter 15 °C gekühlt wird, und
e) die ggf. abschließend sprühgetrocknet wird.

15. Verwendung nach einem der Ansprüche 1 bis 14, wobei eine Getreideschlempe auf Weizen-, Gerste-, Roggen-, Hafer-, Mais- und/oder Reis-Basis eingesetzt wird.

16. Verwendung nach einem der Ansprüche 1 bis 15, wobei die Getreideschlempe in einer Tagesdosis von 100 bis 400 g verabreicht wird.

17. Verwendung nach Anspruch 16, wobei die Getreideschlempe in 2 bis 5 Einzeldosen pro Tag verabreicht wird.

18. Verwendung nach Anspruch 17, wobei die Getreideschlempe in gleichgroßen Einzeldosen verabreicht wird.

19. Verwendung nach einem der Ansprüche 1 bis 18, wobei die Getreideschlempe in viskoser Form verabreicht wird.

20. Verwendung nach Anspruch 19, wobei die Getreideschlempe in Wasser oder fermentierter Milch verabreicht wird.

21. Verwendung nach Anspruch 19, wobei die Getreideschlempe in Sauermilch, Buttermilch, Joghurt, Quark oder Kefir verabreicht wird.

22. Verwendung nach einem der Ansprüche 1 bis 21, wobei die Getreideschlempe als Mahlzeitersatz und/oder als Mahlzeitergänzung verabreicht wird.

23. Verwendung nach Anspruch 22, wobei die Getreideschlempe über einen Zeitraum von mindestens 3 aufeinander folgenden Wochen verabreicht wird.

24. Verwendung nach einem der Ansprüche 1 bis 23, wobei die Getreideschlempe in Kombination und/oder gemeinsam mit weiteren anti-diabetisch wirkenden oder Diabetes vorbeugenden Wirkstoffen eingesetzt wird.

25. Verwendung nach Anspruch 24, wobei die Getreideschlempe in Kombination und/oder gemeinsam mit weiteren dem Diabetes Typ II entgegenwirkenden oder vorbeugenden Wirkstoffen eingesetzt wird.

26. Verwendung nach einem der Ansprüche 24 oder 25, wobei die Getreideschlempe in Form von und/oder in Kombination mit und/oder gemeinsam mit Nahrungsergänzungsmitteln, Functional Food und/oder Spezialernährung eingesetzt wird.

27. Verwendung nach Anspruch 24 bis 26, wobei die Getreideschlempe in Form von und/oder in Kombination mit und/oder gemeinsam mit Kreatin, Kreatin-Monohydrat, α-Liponsäure, Phytosterol, mehrfach ungesättigten Fettsäuren und/oder Phospholipiden sowie deren Derivate und/oder Gemische eingesetzt wird.

28. Verwendung nach einem der Ansprüche 1 bis 27 wobei die Zusammensetzung zusätzlich einem regulierenden Einfluss auf die Darmflora ausübt.

29. Verwendung nach Anspruch 28, wobei die Ballaststoffe der Getreideschlempe diese Wirkung entfalten.

30. Verwendung nach Anspruch 28 oder 29, wobei die Getreideschlempe als Mahlzeitersatz und/oder als Mahlzeitergänzung verabreicht wird.

31. Verwendung nach einem der Ansprüche 28 bis 30, wobei die Getreideschlempe mit ausreichend Flüssigkeit verabreicht wird.

## Claims

1. Use of a distiller's grain which, in addition to being fermented with yeast, has also been fermented with a yoghurt and/or butter culture, in the production of a composition for the prophylaxis and/or treatment of high blood sugar values.

2. Use according to claim 1, wherein the high blood sugar values are selected from fasting and/or post-prandial blood sugar values.

3. Use according to claim 1, wherein a distiller's grain whose residual starch content is not more than 4 wt.%, based on the total weight of the distiller's grain, is used.

4. Use according to any one of claims 1 to 3 in the production of a composition for the prophylaxis and/or treatment of patients with pre-diabetes and/or diabetes and/or a predisposition therefor and forms and/or pre-forms associated therewith.

5. Use according to claim 4, wherein diabetes is selected from type Ia and Ib diabetes, type IIa and IIb diabetes and/or autosomally inherited childhood diabetes, and wherein the forms and/or pre-forms associated therewith are selected from secondary forms of diabetes, potential diabetes, latent diabetes, reduced glucose tolerance and/or clinically manifest diabetes.

6. Use according to claim 5, wherein diabetes is type II diabetes.

7. Use according to any one of claims 1 to 6 further in the production of a composition for reducing body weight.

8. Use according to any one of claims 1 to 7 in the production of a composition for the prophylaxis and/or treatment of patients with obesity.

9. Use according to claim 8 in the production of a composition for the prophylaxis and/or treatment of patients with obesity and at the same time high fasting blood sugar values.

10. Use according to any one of claims 1 to 9 further in the production of a composition for the prophylaxis and/or treatment of patients with metabolic syndrome.

11. Use according to claim 10 in the production of a composition for the prophylaxis and/or treatment of patients with insulin resistance, arteriosclerosis, disorders of lipid, protein and/or carbohydrate metabolism.

12. Use according to any one of claims 1 to 11, wherein the distiller's grain has a low glycaemic index.

13. Use according to any one of claims 1 to 12, wherein the distiller's grain used has been produced by removing the distiller's grain directly from the distillation kettle, then thickening but not drying it, and subsequently fermenting it with milk and a yoghurt and/or butter culture.

14. Use according to claim 13, wherein the distiller's grain used has been produced as follows:
a) the distiller's grain is thickened at a pressure of from 100 to 800 mbar to approximately from 15 to 17 % dry matter, then
b) is mixed with milk that has already been inoculated with the yoghurt and/or butter culture,
c) this mixture either is fermented with a yoghurt culture at a temperature of from 38 to 48°C for from 10 to 20 hours or
is fermented with a butter culture at a temperature of from 18 to 24°C for up to 36 hours, and then
d) is mixed with further milk or concentrated milk and cooled to temperatures below 15°C, and
e) finally is optionally spray-dried.

15. Use according to any one of claims 1 to 14, wherein a distiller's grain based on wheat, barley, rye, oats, maize and/or rice is used.

16. Use according to any one of claims 1 to 15, wherein the distiller's grain is administered in a daily dose of from 100 to 400 g.

17. Use according to claim 16, wherein the distiller's grain is administered in from 2 to 5 individual doses per day.

18. Use according to claim 17, wherein the distiller's grain is administered in individual doses of equal size.

19. Use according to any one of claims 1 to 18, wherein the distiller's grain is administered in viscous form.

20. Use according to claim 19, wherein the distiller's grain is administered in water or fermented milk.

21. Use according to claim 19, wherein the distiller's grain is administered in sour milk, buttermilk, yoghurt, quark or kefir.

22. Use according to any one of claims 1 to 21, wherein the distiller's grain is administered as a meal replacement and/or as a meal supplement.

23. Use according to claim 22, wherein the distiller's grain is administered over a period of at least 3 successive weeks.

24. Use according to any one of claims 1 to 23, wherein the distiller's grain is used in combination and/or together with further active ingredients that have anti-diabetic action and/or that prevent diabetes.

25. Use according to claim 24, wherein the distiller's grain is used in combination and/or together with further active ingredients that counteract or prevent type II diabetes.

26. Use according to either claim 24 or claim 25, wherein the distiller's grain is used in the form of and/or in combination with and/or together with food supplements, functional food and/or special food.

27. Use according to claim 24 to 26, wherein the distiller's grain is used in the form of and/or in combination with and/or together with creatine, creatine monohydrate, α-liponic acid, phytosterol, polyunsaturated fatty acids and/or phospholipids as well as derivatives and/or mixtures thereof.

28. Use according to any one of claims 1 to 27, wherein the composition additionally has a regulating effect on intestinal flora.

29. Use according to claim 28, wherein the roughage in the distiller's grain develops this action.

30. Use according to claim 28 or 29, wherein the distiller's grain is administered as a meal replacement and/or as a meal supplement.

31. Use according to any one of claims 28 to 30, wherein the distiller's grain is administered with sufficient liquid.

## Revendications

1. Utilisation d'une drèche de céréales, qui, en plus d'une fermentation avec une levure, a été soumise à une fermentation avec une culture de yaourt et/ou de beurrerie pour la préparation d'une composition destinée à la prévention et/ou au traitement de taux de glycémie trop élevés.

2. Utilisation selon la revendication 1, dans laquelle les taux de glycémie trop élevés sont choisis parmi les taux de glycémie à jeun ou postprandiaux.

3. Utilisation selon la revendication 1, dans laquelle on utilise une drèche de céréales dont la teneur résiduelle en amidon est au plus de 4 % en masse par rapport à la masse totale de la drèche de céréales.

4. Utilisation selon l'une des revendications 1 à 3 pour la préparation d'une composition destinée à la prévention et/ou au traitement de patients ayant un prédiabète et/ou un diabète et/ou une prédisposition à un diabète et des formes et/ou des formes préliminaires qui y sont associées.

5. Utilisation selon la revendication 4, dans laquelle le diabète est choisi parmi le diabète de type Ia et Ib, de type IIa et IIb et/ou un diabète héréditaire autosomique juvénile, et dans laquelle les formes et/ou les formes préliminaires qui y sont associées sont choisies parmi les formes de diabète secondaires, le diabète potentiel, le diabète latent, une tolérance réduite au glucose et/ou un diabète cliniquement manifeste.

6. Utilisation selon la revendication 5, dans laquelle le diabète est un diabète de type II.

7. Utilisation selon l'une des revendications 1 à 6, pour la préparation en outre d'une composition destinée à réduire la masse corporelle.

8. Utilisation selon l'une des revendications 1 à 7, pour la préparation d'une composition destinée à la prévention et/ou au traitement de patients souffrant d'adipose.

9. Utilisation selon la revendication 8 pour la préparation d'une composition destinée à la prévention et/ou au traitement de patients souffrant d'adipose et ayant simultanément des taux de glycémie à jeun trop élevés.

10. Utilisation selon l'une des revendications 1 à 9 pour la préparation en outre d'une composition destinée à la prévention et/ou au traitement de patients souffrant d'un syndrome métabolique.

11. Utilisation selon la revendication 10 pour la préparation d'une composition destinée à la prévention et/ou au traitement de patients souffrant d'une résistance à l'insuline, d'une artériosclérose, de troubles du métabolisme des lipides, des protéines et/ou des hydrates de carbone.

12. Utilisation selon l'une des revendications 1 à 11, dans laquelle la drèche de céréales présente un faible indice glycémique.

13. Utilisation selon l'une des revendications 1 à 12, dans laquelle on utilise une drèche de céréales qui a été préparée par un procédé dans lequel on prélève la drèche directement de la chaudière, puis on la concentre, mais sans la sécher, et on la met ensuite à fermenter avec du lait et une culture de yaourt et/ou de beurrerie.

14. Utilisation selon la revendication 13, dans laquelle on utilise une drèche de céréales qui a été préparée par un procédé dans lequel
a) on concentre la drèche de céréales sous une pression de 100 à 800 mbar jusqu'à environ 15 à 17 % de masse sèche, puis
b) on mélange avec du lait déjà ensemencé avec la culture de yaourt et/ou de beurrerie,
c) soit on met ce mélange à fermenter avec une culture de yaourt à une température de 38 à 48°C pendant 10 à 20 heures, soit on met ce mélange à fermenter avec une culture de beurrerie à une température de 18 à 24°C pendant jusqu'à 36 heures, puis
d) on mélange avec du lait supplémentaire ou du lait concentré et on refroidit à des températures inférieures à 15°C, et
e) éventuellement, on sèche ensuite par pulvérisation.

15. Utilisation selon l'une des revendications 1 à 14, dans laquelle on utilise une drèche de céréales à base de blé, d'orge, de seigle, d'avoine, de maïs et/ou de riz.

16. Utilisation selon l'une des revendications 1 à 15, dans laquelle la drèche de céréales est administrée à une dose journalière de 100 à 400 g.

17. Utilisation selon la revendication 16, dans laquelle la drèche de céréales est administrée en 2 à 5 doses individuelles par jour.

18. Utilisation selon la revendication 17, dans laquelle la drèche de céréales est administrées en doses individuelles identiques.

19. Utilisation selon l'une des revendications 1 à 18, dans laquelle la drèche de céréales est administrée sous une forme visqueuse.

20. Utilisation selon la revendication 19, dans laquelle la drèche de céréales est administrée dans de l'eau ou du lait fermenté.

21. Utilisation selon la revendication 19, dans laquelle la drèche de céréales est administrée dans du lait caillé, du babeurre, du yaourt, du fromage blanc ou du kéfir.

22. Utilisation selon l'une des revendications 1 à 21, dans laquelle la drèche de céréales est administrée sous forme de succédané de repas et/ou de complément de repas.

23. Utilisation selon la revendication 22, dans laquelle la drèche de céréales est administrée sur une période d'au moins 3 semaines consécutives.

24. Utilisation selon l'une des revendications 1 à 23, dans laquelle la drèche de céréales est utilisée en combinaison et/ou conjointement avec d'autres substances actives antidiabétiques ou prévenant le diabète.

25. Utilisation selon la revendication 24, dans laquelle la drèche de céréales est administrée en combinaison et/ou conjointement avec d'autres substances actives combattant ou prévenant le diabète de type II.

26. Utilisation selon l'une des revendications 24 ou 25, dans laquelle la drèche de céréales est utilisée sous forme de, et/ou en combinaison et/ou conjointement avec des compléments alimentaires, des aliments fonctionnels et/ou une alimentation spéciale.

27. Utilisation selon les revendications 24 à 26, dans laquelle la drèche de céréales est utilisée sous forme de, et/ou en combinaison et/ou conjointement avec de la créatine, du monohydrate de créatine, de l'acide α-lipoïque, du phytostérol, des acides gras polyinsaturés et/ou des phospholipides et leurs dérivés et/ou leurs mélanges.

28. Utilisation selon l'une des revendications 1 à 27, dans laquelle la composition exerce en outre un effet régulateur sur la flore intestinale.

29. Utilisation selon la revendication 28, dans laquelle les fibres de la drèche de céréales développent cette action.

30. Utilisation selon la revendication 28 ou 29, dans laquelle la drèche de céréales est administrée sous forme de succédané de repas et/ou de complément de repas.

31. Utilisation selon l'une des revendications 28 à 30, dans laquelle la drèche de céréales est administrée avec une quantité suffisante de liquide.
